(19) **Europäisches Patentamt**
**European Patent Office**
**Office européen des brevets**

(11) **EP 2 999 782 B1**

(12) **EUROPÄISCHE PATENTSCHRIFT**

(45) Veröffentlichungstag und Bekanntmachung des Hinweises auf die Patenterteilung:
**04.07.2018 Patentblatt 2018/27**

(21) Anmeldenummer: **14723824.0**

(22) Anmeldetag: **15.05.2014**

(51) Int Cl.:
*C12N 9/08* (2006.01)    *C11D 3/386* (2006.01)

(86) Internationale Anmeldenummer:
**PCT/EP2014/059916**

(87) Internationale Veröffentlichungsnummer:
**WO 2014/187719 (27.11.2014 Gazette 2014/48)**

(54) **PEROXIDASEN MIT AKTIVITÄT FÜR CAROTINOIDE**

PEROXIDASES HAVING ACTIVITY FOR CAROTENOIDS

PEROXYDASES POSSÉDANT UNE ACTIVITÉ POUR LES CAROTÉNOÏDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.05.2013 DE 102013209545**

(43) Veröffentlichungstag der Anmeldung:
**30.03.2016 Patentblatt 2016/13**

(73) Patentinhaber: **Henkel AG & Co. KGaA**
**40589 Düsseldorf (DE)**

(72) Erfinder:
- **WEBER, Thomas**
**41541 Dormagen (DE)**
- **MERKEL, Marion**
**51145 Köln (DE)**
- **PRÜSER, Inken**
**40215 Düsseldorf (DE)**
- **MUßMANN, Nina**
**47877 Willich (DE)**
- **O'CONNELL, Timothy**
**40547 Düsseldorf (DE)**
- **HELLMUTH, Hendrik**
**40237 Düsseldorf (DE)**
- **LINKE, Diana**
**31547 Rehburg (DE)**
- **BERGER, Ralf G.**
**30455 Hannover (DE)**

(56) Entgegenhaltungen:
**WO-A1-89/09813    WO-A1-92/18683**

**WO-A1-2005/116180**

- **MOREIRA P R ET AL: "Molecular characterisation of a versatile peroxidase from a Bjerkandera strain", JOURNAL OF BIOTECHNOLOGY, ELSEVIER SCIENCE PUBLISHERS, AMSTERDAM, NL, Bd. 118, Nr. 4, 10. September 2005 (2005-09-10), Seiten 339-352, XP027663347, ISSN: 0168-1656 [gefunden am 2005-09-10]**
- **DATABASE EMBL [Online] 6. September 2005 (2005-09-06), "Bjerkandera adusta versatile peroxidase mRNA, complete cds.", XP002727357, gefunden im EBI accession no. EMBL:DQ060037 Database accession no. DQ060037**
- **MOHORCIC M ET AL: "Expression of soluble versatile peroxidase of Bjerkandera adusta in Escherichia coli", BIORESOURCE TECHNOLOGY, ELSEVIER BV, GB, Bd. 100, Nr. 2, 1. Januar 2009 (2009-01-01), Seiten 851-858, XP025535073, ISSN: 0960-8524, DOI: 10.1016/J.BIORTECH.2008.07.005 [gefunden am 2008-08-15] -& DATABASE EMBL [Online] 23. Mai 2007 (2007-05-23), "Bjerkandera adusta versatile peroxidase (vp) mRNA, partial cds.", XP002727358, gefunden im EBI accession no. EMBL:EF570873 Database accession no. EF570873**
- **ANASTASI A ET AL: "Scale-up of a bioprocess for textile wastewater treatment using Bjerkandera adusta", BIORESOURCE TECHNOLOGY, ELSEVIER BV, GB, Bd. 101, Nr. 9, 1. Mai 2010 (2010-05-01), Seiten 3067-3075, XP026870349, ISSN: 0960-8524 [gefunden am 2010-01-13]**

EP 2 999 782 B1

**Beschreibung**

Die vorliegende Erfindung liegt auf dem Gebiet der Enzymtechnologie. Die Erfindung betrifft Peroxidasen mit Aktivität für Carotinoide, deren Herstellung, alle hinreichend ähnlichen Peroxidasen und für sie codierende Nukleinsäuren sowie Wirtsorganismen, die diese Nukleinsäuren enthalten. Die Erfindung betrifft ferner Verfahren, die diese Peroxidasen verwenden, sowie sie enthaltende Mittel, insbesondere Wasch- und Reinigungsmittel.

Der Einsatz von Enzymen in Wasch- und Reinigungsmitteln ist im Stand der Technik etabliert. Sie dienen dazu, das Leistungsspektrum der betreffenden Mittel entsprechend ihren speziellen Aktivitäten zu erweitern. Hierzu gehören insbesondere hydrolytische Enzyme wie Proteasen, Amylasen, Lipasen und Cellulasen. Die ersten drei genannten hydrolysieren Proteine, Stärke und Fette und tragen somit unmittelbar zur Schmutzentfernung bei. Cellulasen werden insbesondere wegen ihrer Gewebewirkung eingesetzt. Zur Erhöhung der bleichenden Wirkung werden in den Wasch- oder Reinigungsmitteln aber auch Oxidoreduktasen, beispielsweise Oxidasen, Oxygenasen, Katalasen (die bei niedrigen $H_2O_2$-Konzentrationen als Peroxidase reagieren), Peroxidasen, wie Halo-, Chloro-, Bromo-, Lignin-, Glucose- oder Manganperoxidasen, Dioxygenasen oder Laccasen (Phenoloxidasen, Polyphenoloxidasen) verwendet.

Geeignete enzymatische Bleichsysteme sind im Stand der Technik bekannt, beispielsweise aus den internationalen Patentveröffentlichungen WO 98/45398 A1, WO 2004/058955 A2, WO 2005/124012 und WO 2005/056782 A2. Solche enzymatischen Systeme können vorteilhafterweise mit organischen, besonders bevorzugt aromatischen, mit den Enzymen wechselwirkenden Verbindungen kombiniert werden, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Herkömmliche Bleichsysteme auf Percarbonat-, Peroxid- oder Chlorbasis sind in wasserhaltigen Formulierungen, d.h. insbesondere vielen Flüssigformulierungen nicht einsetzbar. Zudem wird der Einsatz solcher Systeme vom Verbraucher im Vergleich zu enzymatischen Systemen als aggressiv und umweltschädlich wahrgenommen. Insofern ist der Einsatz enzymatischer Systeme aus Gründen der Nachhaltigkeit wünschenswert. Enzymatische Bleichsysteme basieren aber üblicherweise ebenfalls auf der enzymatischen Generierung von Wasserstoffperoxid durch den Abbau geeigneter Enzymsubstrate. Diese Substrate müssen den Wasch- oder Reinigungsmitteln zugesetzt werden und stellen einen zusätzlichen Kostenfaktor und teilweise auch einen zusätzlichen toxikologischen oder allergologischen Risikofaktor dar. In flüssigen Einkomponentensystemen stellt sich des Weiteren das Problem, dass Substrat und Enzym bereits vor dem Einsatz in der Wasch- oder Reinigungsflotte in Kontakt kommen und daher ein vorzeitiger Abbau des Substrats aufwendig vermieden werden muss.

Bleichesysteme sind aber zur Entfernung bestimmter stark färbender Anschmutzungen auf Textilien und harten Oberflächen, beispielsweise Carotinoid-enthaltender Anschmutzungen, erforderlich, um eine zufrieden stellende Reinigungsleistung zu erzielen. Carotinoid-haltige Anschmutzungen auf Textilien sind mit handelsüblichen Flüssigwaschmitteln schwer zu entfernen. Darüber hinaus werfen Carotinoid-haltige Anschmutzungen auf Geschirr das Problem auf, dass sie beim maschinellen Geschirrspülen in der Reinigungsflotte verteilt werden und in Kunststoffe eindiffundieren und diese verfärben. Diese Verfärbungen sind dem Anwender bekannt und es ist wünschenswert diese zu verringern.

Es besteht daher Bedarf an substratunabhängigen enzymatischen Systemen, die insbesondere auf Carotinoid-haltigen Anschmutzungen eine Aufhellung bewirken bzw. diese Anschmutzungen entfärben, sowie die Wiederanhaftung von färbenden Anschmutzungen aus der Wasch- oder Reinigungsflotte auf die zu waschenden Gegenstände verhindern.

Um für den Einsatz in Wasch- und Reinigungsmitteln geeignet zu sein, ist es ferner wünschenswert, dass derartige Enzymsysteme eine Enzymaktivität im neutralen bis leicht alkalischen pH-Wert Bereich und in einem breiten Temperaturbereich bis 95 °C, insbesondere im Bereich 30-65 °C aufweisen.

Peroxidasen aus Bjerkandera adusta sind im Stand der Technik bekannt (Moreira et al. J Biotechnol. (2005), 118(4): 339-352; Mohoric et al. Bioresource Techn. (2009), 100(2): 851-858). Es wurde nun eine neue Peroxidase aus Bjerkandera adusta gefunden, die die gewünschten Eigenschaften aufweist und daher besonders gut für den Einsatz in Wasch- und Reinigungsmitteln geeignet ist. Die gefundene Peroxidase besitzt eine deutliche Aktivität auf Carotinoide. Dadurch kann das Enzym auch ohne zusätzliche Substrate zur Aufhellung von Carotinoid-haltigen Anschmutzungen eingesetzt werden.

In einem ersten Aspekt, betrifft die Erfindung daher eine Peroxidase umfassend eine Aminosäuresequenz, die zu der in SEQ ID NO. 1 angegebenen Aminosäuresequenz über deren Gesamtlänge identisch ist. In verschiedenen Ausführungsformen besitzt die Peroxidase eine enzymatische Aktivität für Carotinoide.

Die hierin beschriebenen Enzyme sind vorzugsweise pilzlichen Ursprungs, insbesondere Homologe der Bjerkandera adusta Peroxidase mit der in SEQ ID NO:1 angegebenen Aminosäuresequenz.

Die hierin beschriebenen Peroxidasen weisen eine enzymatische Aktivität auf, das heißt, sie sind befähigt, Carotinoide oxidativ zu spalten. Die oxidative Spaltung ist unabhängig von dem Vorhandensein von Wasserstoffperoxid, kann aber durch die Anwesenheit von Wasserstoffperoxid verstärkt werden. Bei einer hierin beschriebenen Peroxidase handelt es sich vorzugsweise um eine reife (mature) Peroxidase, d.h. um das katalytisch aktive Molekül ohne Signal- und/oder Propeptid(e). Soweit nicht anders angegeben beziehen sich auch die angegebenen Sequenzen auf jeweils reife Enzyme.

Der Begriff "Carotinoide", wie hierin verwendet, bezieht sich auf Verbindungen der Stoffklasse der Terpene, die als natürlichen Farbstoffe, die eine gelbe bis rötliche Färbung verursachen, vorkommen. Es sind etwa 800 verschiedene Carotinoide bekannt, die vor allem in den Chromoplasten und Plastiden der Pflanzen, in Bakterien, aber auch in der Haut, der Schale und im Panzer von Tieren sowie in den Federn und im Eigelb der Vögel vorkommen, wenn die betreffenden Tiere mit ihrer Nahrung farbstoffhaltiges Pflanzenmaterial aufnehmen. Nur Bakterien, Pflanzen und Pilze sind in der Lage, diese Pigmente de novo zu synthetisieren. Carotinoide sind formal aus 8 Isopren-Einheiten aufgebaut und zählen damit zu den Tetraterpenen. Man unterteilt sie in Carotine, die nur aus Kohlenstoff und Wasserstoff aufgebaut sind und Xanthophylle, sauerstoffhaltige Derivate der Carotine. Das Absorptionsspektrum der Carotinoide liegt bei Wellenlängen im Bereich 400 bis 500 Nanometer. Das bekannteste und am häufigsten vorkommende Carotinoid ist das β-Carotin (Karotte), das auch als Provitamin A bekannt ist. Weitere häufig vorkommende Carotinoide sind α-Carotin, Lycopin (Tomate), β-Cryptoxanthin, Capsanthin (rote Paprika), Lutein und Zeaxanthin.

Ferner verfügen bevorzugte Ausführungsformen der Peroxidasen über eine besondere Stabilität in Wasch- oder Reinigungsmitteln, beispielsweise gegenüber Tensiden und/oder Bleichmitteln und/oder gegenüber Temperatureinflüssen, insbesondere gegenüber hohen Temperaturen, insbesondere während des Wasch- bzw. Reinigungsprozesses, beispielsweise zwischen 50 und 65°C, insbesondere 60°C, und/oder gegenüber sauren oder alkalischen Bedingungen und/oder gegenüber pH-Wert-Änderungen und/oder gegenüber denaturierenden oder oxidierenden Agentien und/oder gegenüber proteolytischem Abbau und/oder gegenüber einer Veränderung der Redox-Verhältnisse. Gemäß einer weiteren besonderen Ausführungsform weisen die erfindungsgemäßen Peroxidasen eine gute Lagerstabilität in Waschmittel oder Reinigungsmittelformulierungen, beispielsweise gemessen bei 30 °C und höheren Temperaturen, insbesondere bei 35 °C oder 40 °C auf.

In verschiedenen Ausführungsformen der Erfindung umfasst die Peroxidase eine Aminosäuresequenz, die zu der in SEQ ID NO. 1 angegebenen Aminosäuresequenz über deren Gesamtlänge zu mindestens 98,5%, 98,8 oder 99,0% identisch ist. Zahlenwerte, die hierin ohne Dezimalstellen angegeben sind, beziehen sich jeweils auf den vollen angegebenen Wert mit einer Dezimalstelle. So steht beispielsweise "99%" für "99,0%". Der Ausdruck "ungefähr", in Zusammenhang mit einem Zahlenwert, bezieht sich auf eine Varianz von ±10% bezogen auf den angegebenen Zahlenwert.

In einem weiteren Aspekt betrifft die Erfindung ein Mittel, das dadurch gekennzeichnet ist, dass es eine Peroxidase umfassend eine Aminosäuresequenz, die zu der in SEQ ID NO. 1 angegebenen Aminosäuresequenz über deren Gesamtlänge eine Sequenzidentität von mindestens 70 %, mindestens 75 %, mindestens 80 %, mindestens 85 %, mindestens 90 %, mindestens 95 %, mindestens 96 %, mindestens 97 %, mindestens 98 %, mindestens 98,5 % oder mindestens 99,0 %, bevorzugt mindestens 99,2 %, mindestens 99, 3 %, mindestens 99,4 %, mindestens 99,5 %, bevorzugt mindestens 99,6 %, mindestens 99,7 % oder mindestens 99,8 % aufweist, enthält. Eine solche Peroxidase besitzt eine enzymatische Aktivität für Carotinoide, d.h. ist in der Lage Carotinoide als Substrat zu nutzen und oxidativ zu spalten. Die Aktivität für Carotinoide ist nachweisbar, insofern als dass sie beispielsweise mit dem in den Beispielen beschriebenen Assays messbar und vorzugsweise auch quantifizierbar ist.

Die in dem Mittel verwendeten Enzyme sind vorzugsweise pilzlichen Ursprungs, insbesondere aus Basidiomyceten, besonders bevorzugt Homologe der Bjerkandera adusta Peroxidase mit der in SEQ ID NO:1 angegebenen Aminosäuresequenz. Bevorzugt ist das Mittel ein Wasch- oder Reinigungsmittel einschließlich eines (maschinellen) Geschirrspülmittels. Da hierin beschriebene Peroxidasen vorteilhafte Reinigungsleistungen insbesondere an Carotinoid-haltigen Anschmutzungen aufweisen, sind die Mittel insbesondere zur Entfernung von solchen Carotinoid-haltigen Anschmutzungen geeignet und vorteilhaft. Derartige Mittel enthalten die hierin beschriebenen Peroxidasen in einer Menge von 1 x 10$^{-8}$ bis 1 Gew.-%, 1 x 10$^{-7}$ bis 0,5 Gew.-%, von 0,00001-0,3 Gew.-%, von 0,0001-0,2 Gew.-% und besonders bevorzugt von von 0,001 bis 0,1 Gew.-% Gew.-% jeweils bezogen auf aktives Protein.

Die (Aktiv-)Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee:

A novel method for multiple sequence alignments. J. Mol. Biol. 302, 205-217) oder Programme, die auf diesen Programmen beziehungsweise Algorithmen basieren. In der vorliegenden Patentanmeldung wurden alle Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Die hierin beschriebenen Peroxidasen können im Vergleich zu der in SEQ ID NO:1 angegebenen Sequenz Aminosäureveränderungen, insbesondere Aminosäure-Substitutionen, -Insertionen oder -Deletionen, aufweisen. Solche Peroxidasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Insbesondere kann daneben auch die Produzierbarkeit, die Prozessierung, Sekretion und andere Schritte der Produktion einschließlich des Downstream-Processings durch gezielte genetische Veränderungen, insbesondere durch Mutageneseverfahren verbessert werden.
Ferner können hierin beschriebene Nukleinsäuren in Rekombinationsansätze eingebracht und damit zur Erzeugung völlig neuartiger Peroxidasen oder anderer Polypeptide genutzt werden.

Das Ziel ist es, in die Moleküle gezielte Mutationen wie Substitutionen, Insertionen oder Deletionen einzuführen, um beispielsweise die Leistung der hierin beschriebenen Enzyme zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Peroxidase erhöht und dadurch ihre Leistung verbessert werden.

Ein weiterer hierin offenbarter Gegenstand ist daher eine Peroxidase, die dadurch gekennzeichnet ist, dass sie aus einer Peroxidase wie vorstehend beschrieben als Ausgangsmolekül erhältlich ist durch ein- oder mehrfache konservative Aminosäuresubstitution. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäure-substitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Alternativ oder ergänzend ist die Peroxidase dadurch gekennzeichnet, dass sie aus einer hierin beschriebenen Peroxidase als Ausgangsmolekül erhältlich ist durch Fragmentierung, Fusions-Deletions-, Insertions- oder Substitutionsmutagenese und eine Aminosäuresequenz umfasst, die über eine Länge von mindestens 100, 150, 200, 250, 300, 310, 320, 330, 340, 350, 360 oder 365 zusammenhängenden Aminosäuren mit dem Ausgangsmolekül übereinstimmt.

Unter durch Insertionsmutation erhaltene Proteine sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Unter Fragmenten werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche (unter Umständen nur ein oder mehrere Aminosäuren). So ist es beispielsweise möglich, an den Termini oder in den Loops des Enzyms weitere einzelne Aminosäuren, insbesondere 1, bis zu 2, bis zu 3, bis zu 4, bis zu 5, insbesondere bis zu 10, bevorzugt bis zu 20 Aminosäuren zu deletieren, ohne dass dadurch die enzymatische Aktivität verloren oder vermindert wird. Besonders bevorzugt sind Deletionen von insbesondere 1, bis zu 2, bis zu 3, bis zu 4, bis zu 5, insbesondere bis zu 10, bevorzugt bis zu 20 Aminosäuren auf der N-terminalen Seite des Enzyms und/oder 1, bis zu 2, bis zu 3, bis zu 4, bis zu 5, bevorzugt bis zu 6 Aminosäuren auf der C-terminalen Seite des Enzyms. Dabei wird die enzymatische Aktivität der Peroxidase bevorzugt nicht oder nur in einem geringen Maßen, insbesondere nur bis zu einer Reduktion von 15 % der Aktivität der Peroxidase gemäß SeqID No. 1, reduziert.

Ferner kann durch derartige Fragmentierung, Deletions-, Fusions- Insertions- oder Substitutionsmutagenese beispielsweise auch die Allergenität betreffender Enzyme gesenkt und somit insgesamt ihre Einsetzbarkeit verbessert werden. Vorteilhafterweise behalten die Enzyme auch nach der Mutagenese ihre enzymatische Aktivität, d.h. ihre enzymatische Aktivität entspricht mindestens derjenigen des Ausgangsenzyms, d.h. in einer bevorzugten Ausführungsform beträgt die enzymatische Aktivität mindestens 80, vorzugsweise mindestens 90 % der Aktivität des Ausgangsenzyms. Auch Substitutionen können vorteilhafte Wirkungen zeigen. Sowohl einzelne wie auch mehrere zusammenhängende Aminosäuren können gegen andere Aminosäuren ausgetauscht werden.

Unter chimären oder hybriden Proteinen sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch Shuffling oder Fusionsmutagenese genannt. Der Sinn einer solchen Fusion kann beispielsweise darin bestehen, mithilfe des heranfusionierten Proteinteils eine bestimmte enzymatische Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können. Zur Realisierung der letztgenannten Alternative ist es beispielsweise möglich, posttranslational oder erst nach einem Aufreinigungsschritt durch eine gezielte proteolytische Spaltung eine einzelne chimäre Polypeptidkette in mehrere zu zerlegen. Insbesondere kann auch die Fusionsmutagenese so eingesetzt werden, dass dabei in dem Fachmann bekannter Weise ein Fusionsprotein erzeugt wird, das einen Teil aufweist, der ausgehend von einer hierin beschriebenen Peroxidase als Ausgangsmolekül erhältlich ist durch Fragmentierung, Deletions-, Insertions- oder Substitutionsmutagenese und eine Aminosäuresequenz umfasst, die über eine Länge von mindestens 100, 150, 200, 250, 300, 310, 320, 330, 340, 350, 360 oder 365 zusammenhängenden Aminosäuren mit dem Ausgangsmolekül übereinstimmend ist, und einen weiteren Teil eines anderen Proteins enthält. Bevorzugt wird eine Sequenz von einer oder mehreren anderen Proteine am C-Terminus und/oder am N-Terminus des von der Peroxidase abgeleiteten Teils angehängt.

Beispielsweise geeignet ist die Einführung von zusätzlichen Domänen, bevorzugt aus anderen Proteinen, die die Expression, die Faltung, die Anheftung an bestimmte Substrate oder die Sekretion positiv beeinflussen. Insbesondere kann eine Kohlenhydratbindungsstelle (carbohydrate binding domain, CBD) in dem Fachmann bekannter Weise als zusätzliche Domäne am C- und/oder am N-Terminus des von der Peroxidase abgeleiteten Teils eingefügt werden. Geeignete Domänen sind im Review-Artikel von Boraston, A. B., et al., Biochem. J. (2004), 382, S. 769-781 beschrieben, auf dessen Inhalt hiermit in vollem Umfang Bezug genommen wird. Eine solche Modifikation führt aufgrund eines verbesserten Targetings bei Textilien mit Baumwollanteil zu einer verbesserten Reinigungsleistung in Bezug auf proteinhaltige Flecken.

Ein weiterer hierin offenbarter Gegenstand ist eine zuvor beschriebene Peroxidase, die zusätzlich stabilisiert ist, insbesondere durch eine oder mehrere Mutationen, beispielsweise Substitutionen, oder durch Kopplung an ein Polymer. Denn eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern.

Weitere Möglichkeiten der Stabilisierung sind beispielsweise:

- Veränderung der Bindung von Metallionen oder Kofaktoren, beispielsweise durch Austauschen von einer oder mehreren der an der Bindung beteiligten Aminosäure(n) gegen eine oder mehrere andere Aminosäuren;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken. Die hierin beschriebenen Enzyme können Manganionen als Kofaktoren enthalten und somit sind stabilisierte Varianten unter anderem solche, in denen die Bindung des Mangans verändert ist.

Ein weiterer hierin beschriebener Gegenstand ist eine Peroxidase wie vorstehend beschrieben, die dadurch gekennzeichnet ist, dass sie mindestens eine chemische Modifikation aufweist. Eine Peroxidase mit einer solchen Veränderung wird als Derivat bezeichnet, d.h. die Peroxidase ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise in vivo durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch in vitro durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein hierin beschriebenes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenität und/oder Immunogenität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines hierin beschriebenen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfasst sind deshalb auch alle Präparationen eines hierin beschriebenen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden.

Die vorliegende Erfindung umfasst die oben beschriebenen Peroxidasen und Varianten und Derivate davon sowohl als solche als auch als Bestand eines Mittels, insbesondere eines Wasch- und Reinigungsmittels, wie oben definiert.

Ein weiterer Gegenstand der Erfindung ist eine Nukleinsäure, die für eine hierin beschriebene Peroxidase codiert, insbesondere eine Nukleinsäure die die in SEQ ID NO:2 angegebene Nukleotidsequenz umfasst, sowie ein Vektor enthaltend eine solche Nukleinsäure, insbesondere ein Klonierungsvektor oder ein Expressionsvektor.

Hierbei kann es sich DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesen Erfindungsgegenstand mit eingeschlossen, die eine der vorstehend beschriebenen Peroxidasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei hierin beschriebenen Nukleinsäuren ein oder mehrere Codons durch synonyme Codons ersetzt sein. Dieser Aspekt bezieht sich insbesondere auf die heterologe Expression der hierin beschriebenen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden. Dementsprechend werden von der vorliegenden Erfindung ebenfalls solche Nukleotidsequenzen erfasst, die für die Expression in einem bestimmten Wirtsorganismus codon-optimiert sind. Die Sequenzidentität kann dabei im Vergleich zu der Vorlage niedrig sein, wobei das kodierte Protein aber identisch bleibt.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen.

Unter Vektoren werden im Sinne der vorliegenden Erfindung aus Nukleinsäuren bestehende Elemente verstanden, die als kennzeichnenden Nukleinsäurebereich eine hierin beschriebene Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine hierin beschriebene Nukleinsäure in einen Vektor kloniert. Zu den Vektoren zählen beispielsweise solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer hierin beschriebenen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-$\beta$-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine hierin beschriebene Nukleinsäure oder einen hierin beschriebenen Vektor beinhaltet, insbesondere eine, die die Peroxidase in das die Wirtszelle umgebende Medium sezerniert, wobei die Nukleinsäure heterolog in Bezug auf die Wirtszelle ist. Bevorzugt wird eine hierin beschriebene Nukleinsäure oder ein hierin beschriebener Vektor in einen Mikroorganismus transformiert, der dann eine Wirtszelle darstellt. Die hierin beschriebene Nukleinsäure ist heterolog in Bezug auf den Wirtsorganismus, d.h. ist keine natürlich in dem Wirtsorganimus vorkommende Sequenz. Alternativ können auch einzelne Komponenten, d.h. Nukleinsäure-Teile oder-Fragmente einer hierin beschriebenen Nukleinsäure derart in eine Wirtszelle eingebracht werden, dass die dann resultierende Wirtszelle eine hierin beschriebene Nukleinsäure oder einen hierin beschriebenen Vektor enthält. Dieses Vorgehen eignet sich besonders dann, wenn die Wirtszelle bereits einen oder mehrere Bestandteile einer hierin beschriebenen Nukleinsäure oder eines hierin beschriebenen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte hierin beschriebene Wirtszellen sezernieren das (transgen) exprimierte Protein in das die Wirtszellen umgebende Medium. Ferner können die Peroxidasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Peroxidase funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der hierin beschriebenen Proteine. Ein Beispiel für eine solche Verbindung ist IPTG wie vorstehend beschrieben.

Wirtszellen können prokaryontische oder bakterielle Zellen sein. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise Escherichia coli wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der Actinomycetales besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Hierin beschriebene Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen anwendbar, und führt dazu, dass sich durch den Einsatz solcher Mikroorganismen hierin beschriebene Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von Escherichia, Klebsiella, Bacillus, Staphylococcus, Corynebakterium, Arthrobacter, Streptomyces, Stenotrophomonas und Pseudomonas, weiter bevorzugt eines, das ausgewählt ist aus der Gruppe von Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia.

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryontischen Zellen sind eukaryontische Zellen in der Lage, das gebildete Protein posttranslational zu modifizieren. Beispiele dafür sind Pilze wie Basidiomyceten, Actinomyceten oder Hefen wie Saccharomyces, Pichia, Hansenula oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen oder Lipasen, kann vorteilhaft sein. Ferner können sich beispielsweise thermophile pilzliche Expressionssysteme besonders zur Expression temperaturbeständiger Proteine oder Varianten eignen. Pilzliche Expressionssysteme sind im Rahmen der Erfindung bevorzugt.

Die Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Die hierin beschriebenen Wirtszellen werden bevorzugt verwendet, um die hierin beschriebenen Peroxidasen herzustellen. Ein weiterer Gegenstand der Erfindung ist daher ein Verfahren zur Herstellung einer Peroxidase umfassend

a) Kultivieren einer hierin beschriebenen Wirtszelle
b) Isolieren der Peroxidase aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der hierin beschriebenen Peroxidase. Alle Fermentationsverfahren, die auf einem entsprechenden Verfahren zur Herstellung einer hierin beschriebenen Peroxidase beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als

auch in der Zellmasse beziehungsweise Trockenmasse und/oder insbesondere in der Aktivität der interessierenden Peroxidase erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Peroxidase kann aus dem Fermentationsmedium geerntet werden. Ein solches Fermentationsverfahren ist gegenüber einer Isolation der Peroxidase aus der Wirtszelle, d.h. einer Produktaufbereitung aus der Zellmasse (Trockenmasse) bevorzugt, erfordert jedoch die Bereitstellung von geeigneten Wirtszellen oder von einem oder mehreren geeigneten Sekretionsmarkern oder -mechanismen und/oder Transportsystemen, damit die Wirtszellen die Peroxidase in das Fermentationsmedium sezernieren. Ohne Sekretion kann alternativ die Isolation der Peroxidase aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um hierin beschriebene Peroxidasen herzustellen.

In den hierin beschriebenen Mitteln, insbesondere Wasch- und Reinigungsmitteln, können die einzusetzenden Enzyme zusammen mit Begleitstoffen, etwa aus der Fermentation, oder mit Stabilisatoren konfektioniert sein. In flüssigen Formulierungen werden die Enzyme bevorzugt als Enzymflüssigformulierung(en) eingesetzt.

Die Peroxidasen können besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung ist eine Inhibierung der Proteolyse besonders bevorzugt. Die beschriebenen Mittel können zu diesem Zweck Stabilisatoren enthalten.

Reinigungsaktive Peroxidasen werden in der Regel nicht in Form des reinen Proteins sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluidbed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Wie aus der vorherigen Ausführungen ersichtlich, bildet das Enzym-Protein nur einen Bruchteil des Gesamtgewichts üblicher Enzym-Zubereitungen. Bevorzugt eingesetzte Peroxidase-Zubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 10 Gew.-% des Enzymproteins.

Die hierin beschriebenen Mittel umfassen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Ein hierin beschriebenes Mittel enthält die Peroxidase vorteilhafterweise in einer Menge von $2\mu g$ bis 20mg, vorzugsweise von $5\mu g$ bis 17,5mg, besonders bevorzugt von $20\mu g$ bis 15mg und ganz besonders bevorzugt von $50\mu g$ bis 10mg pro g des Mittels. Ferner kann die in dem Mittel enthaltene Peroxidase, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Peroxidase mit einer bei Raumtemperatur oder bei Abwesenheit von Wasser für die Peroxidase undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

Diese Ausführungsformen der vorliegenden Erfindung umfassen alle festen, pulverförmigen, flüssigen, gelförmigen oder pastösen Darreichungsformen hierin beschriebener Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwasch- oder -geschirrspülmittels oder einer nicht-wässrigen Paste oder in Form eines wässrigen Flüssigwasch- oder -geschirrspülmittels oder einer wasserhaltigen Paste. Auch ein flüssiges, gelförmiges oder pastöses Mittel kann in einer wasserlöslichen Umhüllung darbeboten werden, welche sich in der Anwendung des Produktes in Wasser auflöst, beispielsweise eingeschweißt in eine Polyvinylakkohol-Folie. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Hierin beschriebene Wasch- oder Reinigungsmittel können zusätzlich zu der hierin beschriebenen Peroxidase auch hydrolytische Enzyme oder andere Enzyme in einer für die Wirksamkeit des Mittels zweckmäßigen Konzentration enthalten. Die Enzyme können in Form der oben beschriebenen Enzymformulierungen vorliegen. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem hierin beschriebenen Mittel eine katalytische Aktivität entfalten können, insbesondere eine Protease, Amylase, Cellulase, Hemicellulase, Mannanase, Tannase, Xylanase, Xanthanase, Xanthanlyase, Xyloglucanase, β-Glucosidase, Pektinase, Pectatlyase, Carrageenase, Perhydrolase, Oxidase, Oxidoreduktase, Cutinase oder eine Lipase, sowie deren Gemische. Weitere Enzyme sind in dem Mittel vorteilhafterweise jeweils in einer Menge von $1 \times 10^{-8}$ bis 5 Gewichts-Prozent bezogen auf aktives Protein enthalten. Zunehmend bevorzugt ist jedes weitere Enzym in einer Menge von $1 \times 10^{-7}$-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in hierin beschriebenen Mitteln enthalten, bezogen auf aktives Protein.

Die hierin beschriebenen Wasch- oder Reinigungsmittel, die als pulverförmige Feststoffe, in nachverdichteter Teilchenform, als homogene Lösungen oder Suspensionen vorliegen können, können neben einer hierin beschriebenen Peroxidase alle bekannten und in derartigen Mitteln üblichen Inhaltsstoffe enthalten, wobei bevorzugt mindestens ein weiterer Inhaltsstoff in dem Mittel vorhanden ist. Die hierin beschriebenen Mittel können insbesondere Tenside, Builder (Gerüststoffe), andere Bleichmittel oder Bleichaktivatoren enthalten. Ferner können sie wassermischbare organische Lösungsmittel, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten. In verschiedenen Ausführungsformen der Erfindung enthalten die hierin beschriebenen Mittel eine Wasserstoffperoxidquelle, beispielsweise ein Percarbonat, Peroxid oder Perborat. Das aus dieser Quelle stammende Wasserstoffperoxid kann die katalytische Aktivität der hierin beschriebenen Peroxidasen weiter steigern. Es ist allerdings bevorzugt, dass die hierin beschriebenen Enzyme auch in Abwesenheit von Wasserstoffperoxid eine oxidative Spaltung von Carotinoide bewirken können.

Vorteilhafte Inhaltsstoffe hierin beschriebener Mittel sind offenbart in der internationalen Patentanmeldung WO2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Ein weiterer Erfindungsgegenstand ist ein Verfahren zur Reinigung von Textilien oder harten Oberflächen, das dadurch gekennzeichnet ist, dass in mindestens einem Verfahrensschritt ein hierin beschriebenes Mittel angewendet wird, oder dass in mindestens einem Verfahrensschritt eine hierin beschriebene Peroxidase katalytisch aktiv wird, insbesondere derart, dass die Peroxidase in einer Menge von 40μg bis 4g, vorzugsweise von 50μg bis 3g, besonders bevorzugt von 100μg bis 2g und ganz besonders bevorzugt von 200μg bis 1g eingesetzt wird.

Hierunter fallen sowohl manuelle als auch maschinelle Verfahren, wobei maschinelle Verfahren bevorzugt sind. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines hierin beschriebenen Wasch- oder Reinigungsmittels oder einer hierin beschriebenen Peroxidase bereichert werden und stellen dann Ausführungsformen der vorliegenden Erfindung dar. Alle Sachverhalte, Gegenstände und Ausführungsformen, die für hierin beschriebene Peroxidasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden beschriebenen Verfahren gilt.

Ausführungsformen dieses Erfindungsgegenstandes stellen auch Verfahren zur Behandlung von Textilrohstoffen oder zur Textilpflege dar, bei denen in wenigstens einem Verfahrensschritt eine hierin beschriebene Peroxidase aktiv wird. Hierunter sind Verfahren für Textilrohstoffe, Fasern oder Textilien mit natürlichen Bestandteilen bevorzugt, und ganz besonders für solche mit Wolle oder Seide.

Ein weiterer Erfindungsgegenstand ist die Verwendung eines hierin beschriebenen Mittels zur Reinigung von Textilien oder von harten Oberflächen, oder einer hierin beschriebenen Peroxidase zur Reinigung von Textilien oder von harten Oberflächen, insbesondere derart, dass die Peroxidase in einer Menge von 40μg bis 4g, vorzugsweise von 50μg bis 3g, besonders bevorzugt von 100μg bis 2g und ganz besonders bevorzugt von 200μg bis 1g eingesetzt wird.

Eine typische Rahmenrezeptur für ein vorzugsweise einsetzbares maschinelles Geschirrspülmittel, beispielsweise in Tablettenform, umfasst folgende Stoffe:

| | |
|---|---|
| Na-Tripolyphosphat | 20-50 Gew.-% |
| Natriumcarbonat | 10-30 Gew.-% |
| Natriumpercarbonat | 5-18 Gew.-% |
| Bleichaktivator | 0,5-5 Gew.-% |
| Bleichkatalysator | 0,01-1 Gew.-% |
| Sulfopolymer | 2,5-15 Gew.-% |
| Polycarboxylat | 0,1-10 Gew.-% |
| Niotensid | 0,5-10 Gew.-% |
| Phosphonat | 0,5-5 Gew.-% |
| Proteasen | 0,1-5 Gew.-% |
| Amylase | 0,1-5 Gew.-%, |

wobei sich die Angabe in Gew.-% jeweils auf das gesamte Mittel beziehen. Statt des oder eines Teils des Tripolyphosphats kann in der Rezeptur insbesondere auch 10-50 Gew.-% Citrat oder MGDA oder GLDA oder EDDS oder Mischungen aus zwei oder drei dieser Substanzen eingesetzt werden.

Alle Sachverhalte, Gegenstände und Ausführungsformen, die für hierin beschriebene Peroxidasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehend beschriebene Verwendung gilt.

Beispiele

Alle molekularbiologischen Arbeitsschritte folgen Standardmethoden. Enzyme und Baukästen (Kits) wurden nach den Angaben der jeweiligen Hersteller eingesetzt.

Beispiel 1: Herstellung, Aufreinigung und Aktivitätsmessung einer B. adusta Peroxidase Vor der Verwendung wurden alle Medien und Laborgegenstände sterilisiert (autoklaviert). Stammkulturen von Bjerkandera adusta wurden in 2 L SNL Medium (30.0 g L$^{-1}$ Glucose Monohydrat; 4.5 g L$^{-1}$ $L$-Asparagin Monohydrat; 1.5 g L$^{-1}$ $KH_2PO_4$; 0.5 g L$^{-1}$ $MgSO_4$; 3.0 g L$^{-1}$ Hefeextrakt; 1.0 mL L$^{-1}$ Spurenelementlösung enthaltend 0.005 g L$^{-1}$ $CuSO_4$ x 5 $H_2O$, 0.08 g L$^{-1}$ $FeCl_3$ x 6 $H_2O$, 0.09 g L$^{-1}$ $ZnSO_4$ x 7 $H_2O$, 0.03 g L$^{-1}$ $MnSO_4$ x $H_2O$ und 0.4 g L$^{-1}$ EDTA) mit 0,01 % Entschäumer bei 24°C, 1 L/min Sauerstoff und 200 rpm kultiviert.

200 mL einer 7 Tage alten Vorkultur wurden mittels eines Ultra-Turrax homogenisiert und dienten zum Animpfen einer Hauptkultur. Es wurden täglich Proben des Überstands entnommen und mittels des unten beschriebenen Assays auf ihre Carotinoid-abbauende Aktivität hin untersucht. Der Überstand wurde zu einem Zeitpunkt an dem ausreichende Aktivität nachgewiesen werden konnte geerntet und mittels Anionenaustauschchromatographie gereinigt. Aktive Fraktionen wurden vereinigt, mit Aktivkohle teilweise entfärbt und mittels Ultrafiltration konzentriert.

Photometrischer Aktivitätsassay

Für die photometrische Bestimmung der Carotin-abbauenden Aktivität wurde 10fach konzentrierter Überstand verwendet. Abhängig von der Aktivität und dem als Substrat verwendeten Carotinoid wurden 10-100 μl der Probe und Wasser/Puffer gemischt, um 1,6 mL zu ergeben. Die Mischung wurde auf 30°C erwärmt und die Reaktion durch Zugabe des Substrats gestartet. Die Verringerung der Extinktion am Absorptionsmaximum wurde über 10 Minuten gemessen und die Enzymaktivität nach folgender Formel berechnet:

$$A \text{ [mU/mL]} = \Delta E \times 1{,}7 \times 1000000 \times F / (\varepsilon \times 1{,}6)$$

$\Delta E$ = Verringerung der Extinktion am Absorptionsmaximum pro minute
$\varepsilon$ = Extinktionskoeffizient in L x mol$^{-1}$ x cm$^{-1}$
F = Verdünnungsfaktor der Probe

Es wurden folgende Carotinoide als Substrat getestet:

Tabelle 1:

| Carotinoid | Absorptionsmaximum (nm) | Extinktionskoeffizient (L x mol$^{-1}$ x cm$^{-1}$) |
|---|---|---|
| Carotin | 460 | 87300 |
| Lycopin | 483 | 114600 |
| Lutein | 457 | 66900 |
| Capsanthin | 478 | 81900 |

Reinigung der Peroxidase

Für die Aufreinigung der Peroxidase wurde eine Q-Sepharose Säule (GE Healthcare, 1 mL) und 20mM Natriumacetat-Puffer pH 5 (ohne und mit 1 M Natriumchlorid) verwendet. 10 mL des Überstands wurden 1:1 mit Laufpuffer gemischt. Die Trennung wurde mit einer Flussrate von 2 mL/min mit einem linearen Gradienten (Laufpuffer + 1 M NaCl) für 15 mL durchgeführt. Es wurden 1 mL Fraktionen gesammelt und mittels des oben beschriebenen Assays auf ihre enzymatische Aktivität getestet.

Beispiel 2: Aufhellung einer Maschinengeschirrspülflotte

Als Beispiel für eine Carotinoid-belastete Maschinengeschirrspülmittel(MGSM)-Spülflotte wird 10 g handelsübliches Tomatenmark (2-fach konzentriert) mit einem enzymhaltigen handelsüblichen Geschirrspülmittel gemäß Tabelle 2 und entionisiertem Wasser auf 200 mL gebracht. Der pH wird nach dem Auflösen vor dem endgültigen Auffüllen auf 200 mL mit Natronlauge auf 9,18 adjustiert.

Tabelle 2: Zusammensetzung des maschinellen Geschirrspülmittels

| | Basis |
|---|---|
| Phosphat (Gew.-%) | 35,9 |
| Natriumcarbonat (Gew.-%) | 12,2 |
| Phosphonat (Gew.-%) | 2,4 |
| Sulfonsäuregruppen-haltiges Polymer (Gew.-%) | 7,9 |
| Polyacrylat (Gew.-%) | 4,6 |
| Nichtionische Tenside (Gew.-%) | 6,1 |
| Percarbonat (Gew.-%) | 14,6 |
| TAED (Gew.-%) | 2,3 |
| Bleichkatalysator (Gew.-%) | 1,0 |
| Polycarboxylat (Gew.-%) | 1,5 |
| Natriumsilikat/Polycarboxylat (Gew.%) | 3,9 |
| Enzymzusammensetzung (Amylase) (Gew.-%) | 1,0 |
| Zinkacetat (Gew.-%) | 0,2 |

(fortgesetzt)

| | Basis |
|---|---|
| Reste (Parfüm, Farbstoffe, Protease bzw. Proteasemischung etc.) (Gew.-%) | ad 100 |

Es werden zwei Ansätze getestet:

Enzymwert: 9 mL der o.g. Spülflotte mit 1 mL Enzympräparation aus Bsp. 1
Leerwert: : 9 mL der o.g. Spülflotte mit 1 mL entionisiertem Wasser.

Beide Ansätze werden in verschlossenen 14 mL Polypropylenröhrchen in einem Rotationsmischer, welcher sich unter einer auf 50°C temperierten Heizhaube befindet, etwa im Sekundentakt kopfüber- und zurückgedreht. Nach 0h, 4h, 21h und 25 h wird je eine 1 mL-Probe entnommen, in einer Tischzentrifuge von Trübstoffen geklärt und der Überstand zur Extinktionsmessung gegen Luft bei 500 nm verwendet.

Die Differenz des Enzymwertes (die "Probendifferenz") zum Leerwert wird für jeden Zeitpunkt bestimmt (negative Werte spiegeln eine geringere Extinktion des Enzymwertes und damit eine hellere Probe wieder). Die zeitabhängige "enzymatische Aufhellung" wird berechnet durch Bildung der Differenz von Probendifferenz des Zeitpunktes x zur Probendifferenz des Startwertes (0h) (negative enzymatische Aufhellungen spiegeln eine Abnahme der Extinktion und somit eine stärkere Aufhellung durch die Enzymprobe wieder).

Tabelle 3:

| Zeit (h): | 0 | 4 | 21 | 25 |
|---|---|---|---|---|
| Enzymatische Aufhellung | 0 | -0,114 | -0,315 | -0,387 |

Die Ergebnisse belegen eine mit der Zeit zunehmende Aufhellung der Spülflotte durch das Enzym.

Beispiel 3: Miniwaschversuch (Flüssigwaschmittel, Tomate & Karotte)

Ein Enzympräparat aus dem Kulturüberstand von B. adusta mit einer CDA (carotenoid degrading activity = Carotinoid abbauenden Aktivität) von 25 U/L wurde hergestellt und in folgendem Waschversuch eingesetzt:
In eine 48 well Mikrotiterplatte wurden rund ausgestanzte (Durchmesser 1cm) Anschmutzungen einzeln vorgelegt (WfK 10O (Baumwolle mit Karottensaft-Anschmutzung) und WfK 10SG (Baumwolle mit Tomatenrindfleischsauce-Anschmutzung).

Auf jedes Läppchen wurde 1000 µL auf 40°C vortemperierte Waschlauge eines Flüssigwaschmittels mit der im Weiteren angegebenen Zusammensetzung pipettiert (Endkonzentration im Versuch 4,7 g/L, 16°dH) und 20 µL der zu testenden Enzymlösung zugegeben. Die Versuche wurden in Triplikaten durchgeführt.

Als Waschmittel-Basis-Rezeptur diente ein Flüssigwaschmittel folgender Zusammensetzung (alle Angaben in Gewichts-Prozent):
0,3-0,5% Xanthan, 0,2-0,4% Anti-Schaummittel, 6-7% Glycerin, 0,3-0,5% Ethanol, 4-7% FAEOS (Fettalkoholethersulfat), 24-28% nichtionische Tenside, 1% Borsäure, 1-2% Natriumcitrat (Dihydrat), 2-4% Soda, 14-16% Kokosnuss-Fettsäuren, 0,5% HEDP (1-Hydroxyethan-1,1-diphosphonsäure), 0-0,4% PVP (Polyvinylpyrrolidon), 0-0,05% optischer Aufheller, 0-0,001% Farbstoff, Rest demineralisiertes Wasser.

Die Platten wurden mit dem zugehörigen Deckel luftdurchlässig verschlossen und für 1, 4 bzw. 16 Stunden im Dunklen auf einem Titramax Inkubationsschüttler bei 40°C gewaschen.

Anschließend wurde die Waschlauge durch ein Sieb abgegossen, dreimal mit Leitungswasser und dreimal mit entionisiertem Wasser gespült, Restwasser durch Tupfen mit Laborpapier vorsichtig abgesogen und 24 oder 48 Stunden bei Raumtemperatur im Dunklen getrocknet. Nach Aufkleben auf weißes Papier wurde mit einem Minolta Farbmeßgerät Helligkeit und Farbe im Vergleich zu Weiß- und Schwarzstandard des Gerätes gemessen.

Zur Bewertung der Aufhellung wurde die Differenz des Helligkeitswertes L* im L*a*b*-System der enzymbehandelten Probe zur gleich behandelten, enzymfreien Probe (x=0) (Mittelwerte der Triplikate) für jede Anschmutzung berechnet ("ΔL*"). In folgender Tabelle ist die Aufhellung nach vierstündiger Behandlung dargestellt (größere Werte sprechen für stärkere Aufhellung der Probe):

Tabelle 4:

| Gewebe | ∆L* |
|--------|-----|
| WfK 10O | 0,2 |
| WfK 10SG | 4,8 |
| Summe | 5,0 |

Die Enzymbehandlung bewirkt eine deutliche Aufhellung gegenüber der enzymfreien Kontrolle, die auch mit dem Auge sichtbar ist.

Ergänzend wurden zur Bewertung der Veränderung der Farbintensität (Entfärbung) für einen weiteren analog durchgeführten Waschversuch die Vektoraddition der drei Farbkomponenten $\sqrt{\Delta L*^2 + \Delta a*^2 + \Delta b*^2}$ errechnet aus den Differenzen der Farbwerte ∆L* und in analoger Weise ∆a* und ∆b*, zwischen dem Testansatz mit und der Kontrolle ohne das Enzym. Hierdurch wird die stark mit dem Auge sichtbare Farbverschiebung vom Orange-gelben hin zum Farbneutralen besser wiedergegeben als nur durch die Aufhellung ∆L* allein.

Die folgende Tabelle stellt die einzelnen Beiträge durch die beiden untersuchten Anschmutzungen zur Summe der so errechneten Entfärbung in diesem Experiment dar (größere Werte bedeuten bessere Entfärbung):

| Entfärbung | 1 Std. | 4 Std. |
|------------|--------|--------|
| WfK 10O | 0,8 | 1,9 |
| WfK 10SG | 4,2 | 11,3 |
| Summe | 5,0 | 13,2 |

Die Ergebnisse belegen eine vorteilhafte Entfärbung der Flecken durch das Enzym. Die Wirkung nimmt mit der Waschzeit zu.

**Patentansprüche**

1. Peroxidase umfassend eine Aminosäuresequenz, die mit der in SEQ ID NO: 1 angegebenen Aminosäuresequenz über deren Gesamtlänge identisch ist.

2. Nukleinsäure codierend für eine Peroxidase nach Anspruch 1, vorzugsweise umfassend die in SEQ ID NO:2 angegebene Nukleotidsequenz.

3. Vektor enthaltend eine Nukleinsäure nach Anspruch 2, insbesondere ein Klonierungsvektor oder ein Expressionsvektor.

4. Nicht menschliche Wirtszelle, insbesondere Pilzzelle, die eine Nukleinsäure nach Anspruch 2 oder einen Vektor nach Anspruch 3 beinhaltet, insbesondere eine, die die Peroxidase in das die Wirtszelle umgebende Medium sezerniert, wobei die Nukleinsäure heterolog in Bezug auf die Wirtszelle ist.

5. Verfahren zur Herstellung einer Peroxidase umfassend

a) Kultivieren einer Wirtszelle gemäß Anspruch 4
b) Isolieren der Peroxidase aus dem Kulturmedium oder aus der Wirtszelle.

6. Wasch- oder Reinigungsmittel, **dadurch gekennzeichnet, dass** es mindestens eine Peroxidase umfassend eine Aminosäuresequenz, die mindestens 70 %, mindestens 75 %, mindestens 80 %, mindestens 85 %, mindestens 90 %, mindestens 95 %, mindestens 96 %, mindestens 97 %, mindestens 98 %, mindestens 98,5 % oder mindestens 99 % Sequenzidentität mit der in SEQ ID NO: 1 angegebenen Aminosäuresequenz über deren Gesamtlänge aufweist, enthält, wobei die Peroxidase enzymatische Aktivität für Carotinoide besitzt.

7. Mittel nach Anspruch 6, **dadurch gekennzeichnet, dass**

   (i) die Peroxidase aus einer Peroxidase mit der in SEQ ID NO:1 angegebenen Aminosäuresequenz als Ausgangsmolekül erhältlich ist durch ein- oder mehrfache konservative Aminosäuresubstitution; und/oder
   (ii) die Peroxidase aus einer Peroxidase mit der in SEQ ID NO:1 angegebenen Aminosäuresequenz als Ausgangsmolekül erhältlich ist durch Fragmentierung, Fusions-Deletions-, Insertions- oder Substitutionsmutagenese und eine Aminosäuresequenz umfasst, die über eine Länge von mindestens 100, 150, 200, 250, 300, 310, 320, 330, 340, 350, 360 oder 365 zusammenhängenden Aminosäuren mit dem Ausgangsmolekül übereinstimmt.

8. Mittel nach Anspruch 6 oder 7, **dadurch gekennzeichnet, dass** das Mittel

   (i) ein flüssiges, wasserhaltiges Wasch- oder Reinigungsmittel, insbesondere Wasch- oder Geschirrspülmittel ist; und/oder
   (ii) zusätzlich eine Wasserstoffperoxidquelle, beispielsweise ein Percarbonat, Peroxid oder Perborat enthält; und/oder
   (iii) zusätzlich Tenside, Builder (Gerüststoffe), von der Peroxidase verschiedene Enzyme, Bleichmittel, Bleichaktivatoren, wassermischbare organische Lösungsmittel, Sequestrierungsmittel, Elektrolyte, pH-Regulatoren und/oder weitere Hilfsstoffe wie optische Aufheller, Vergrauungsinhibitoren, Schaumregulatoren, sowie Farb- und Duftstoffe sowie Kombinationen hiervon enthält.

9. Ausführungsform nach einem der Ansprüche 1-8, **dadurch gekennzeichnet, dass** die Peroxidase in einem photometrischen Aktivitätsassay nachweisbare Aktivität für Carotinoide als Substrat aufweist.

10. Verfahren zur Reinigung von Textilien oder harten Oberflächen, **dadurch gekennzeichnet, dass** in mindestens einem Verfahrensschritt ein Mittel nach einem der Ansprüche 6-8 angewendet wird.


**Claims**

1. A peroxidase comprising an amino acid sequence which is identical to the amino acid sequence shown in SEQ ID NO: 1 over the total length thereof.

2. A nucleic acid which codes for a peroxidase according to claim 1, preferably comprising the nucleotide sequence shown in SEQ ID NO: 2.

3. A vector containing a nucleic acid according to claim 2, in particular a cloning vector or an expression vector.

4. A non-human host cell, in particular a fungal cell, containing a nucleic acid according to claim 2 or a vector according to claim 3, in particular a non-human host cell which secretes the peroxidase into the medium surrounding the host cell, wherein the nucleic acid is heterologous with regard to the host cell.

5. A method for preparing a peroxidase comprising

   a) cultivating a host cell according to claim 4
   b) isolating the peroxidase from the culture medium or from the host cell.

6. A washing or cleaning agent, **characterized in that** it contains a peroxidase comprising an amino acid sequence, which is at least 70%, at least 75%, at least 80%, at least 85%, at least 90%, at least 95%, at least 96%, at least 97%, at least 98%, at least 98.5% or at least 99% identical to the amino acid sequence shown in SEQ ID NO: 1 over the total length thereof, the peroxidase having enzymatic activity for carotenoids.

**7.** The agent according to claim 6, **characterized in that**

(i) the peroxidase can be obtained as a starter molecule from a peroxidase having the amino acid sequence shown in SEQ ID NO: 1 by means of single or multiple conservative amino acid substitutions; and/or

(ii) the peroxidase can be obtained as a starter molecule from a peroxidase having the amino acid sequence shown in SEQ ID NO: 1 by means of fragmentation or fusion, deletion, insertion or substitution mutagenesis, and comprises an amino acid sequence which matches the starter molecule over a length of at least 100, 150, 200, 250, 300, 310, 320, 330, 340, 350, 360, or 365 interconnected amino acids.

**8.** The agent according to claim 6 or 7, **characterized in that** the agent

(i) is a liquid, aqueous washing or cleaning agent, in particular a washing or dishwashing detergent; and/or

(ii) additionally contains a hydrogen peroxide source, for example a percarbonate, peroxide or perborate; and/or

(iii) additionally contains surfactants, builders, enzymes that are different from the peroxidase, bleaching agents, bleach activators, water-miscible organic solvents, sequestering agents, electrolytes, pH regulators and/or further auxiliaries such as optical brighteners, graying inhibitors, foam regulators, and dyes and fragrances, and combinations thereof.

**9.** An embodiment according to one of claims 1-8, **characterized in that** the peroxidase has, as a substrate, detectable activity for carotenoids in a photometric activity assay.

**10.** A method for cleaning textiles or hard surfaces, **characterized in that** an agent according to one of claims 6-8 is used in at least one method step.

## Revendications

**1.** Peroxydase comprenant une séquence d'acides aminés, identique sur toute sa longueur à la séquence d'acides aminés indiquée dans SEQ ID NO : 1.

**2.** Acide nucléique codant pour une peroxydase selon la revendication 1, de préférence comprenant la séquence de nucléotides indiquée dans SEQ ID NO : 2.

**3.** Vecteur contenant un acide nucléique selon la revendication 2, en particulier un vecteur de clonage ou un vecteur d'expression.

**4.** Cellule hôte non-humaine, en particulier cellule fongique, contenant un acide nucléique selon la revendication 2 ou un vecteur selon la revendication 3, en particulier une cellule sécrétant la peroxydase dans le milieu entourant la cellule hôte, dans laquelle l'acide nucléique est hétérologue par rapport à la cellule hôte.

**5.** Procédé de synthèse d'une peroxydase comprenant

a) la culture d'une cellule hôte selon la revendication 4
b) l'isolement de la peroxydase du milieu de culture ou de la cellule hôte.

**6.** Agent nettoyant ou détergent, **caractérisé en ce qu'**il contient au moins une peroxydase comprenant une séquence d'acides aminés, cette séquence ayant une identité de séquence sur toute sa longueur d'au moins 70 %, d'au moins 75 %, d'au moins 80 %, d'au moins 85 %, d'au moins 90 %, d'au moins 95 %, d'au moins 96 %, d'au moins 97 %, d'au moins 98 %, d'au moins 98,5 % ou d'au moins 99 % avec la séquence d'acides aminés indiquée dans SEQ ID NO : 1, la peroxydase possédant une activité enzymatique pour les caroténoïdes.

**7.** Agent selon la revendication 6, **caractérisé en ce que**

(i) la peroxydase peut être obtenue à partir d'une peroxydase ayant pour molécule mère la séquence d'acides aminés indiquée dans SEQ ID NO : 1 au moyen d'une substitution conservatrice simple ou multiple d'acides aminés ; et/ou

(ii) la peroxydase peut être obtenue à partir d'une peroxydase ayant pour molécule mère la séquence d'acides aminés indiquée dans SEQ ID NO : 1 au moyen d'une fragmentation, d'une mutagénèse de fusion, de délétion, d'insertion ou de substitution et comprend une séquence d'acides aminés concordant avec la molécule mère sur une longueur d'au moins 100, 150, 200, 250, 300, 310, 320, 330, 340, 350, 360 ou 365 acides aminés consécutifs.

8. Agent selon la revendication 6 ou 7, **caractérisé en ce que** l'agent

    (i) est un agent nettoyant ou détergent liquide aqueux, en particulier une lessive ou un liquide vaisselle ; et/ou
    (ii) contient en outre une source de peroxyde d'hydrogène, par exemple un percarbonate, un peroxyde ou un perborate ; et/ou
    (iii) contient en outre des tensioactifs, des builders (édificateurs), des enzymes différentes de la peroxydase, des agents de blanchiment, des activateurs de blanchiment, des solvants organiques miscibles dans l'eau, des séquestrants, des électrolytes, des régulateurs de pH et/ou d'autres adjuvants tels que des azurants optiques, des agents antiredéposition, des régulateurs de mousse, ainsi que des colorants et des parfums ou des combinaisons de ceux-ci.

9. Mode de réalisation selon l'une des revendications 1 à 8, **caractérisé en ce que** la peroxydase comporte, lors d'un essai photométrique d'activité, une activité pouvant être attestée pour les caroténoïdes en tant que substrat.

10. Procédé de nettoyage de textiles ou de surfaces dures, **caractérisé en ce qu'**un agent selon l'une des revendications 6 à 8 est appliqué dans au moins une étape du procédé.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9845398 A1
- WO 2004058955 A2
- WO 2005124012 A
- WO 2005056782 A2
- WO 2009121725 A
- PT 031371
- PT 102013209545

**In der Beschreibung aufgeführte Nicht-Patentliteratur**

- **MOREIRA et al.** *J Biotechnol.,* 2005, vol. 118 (4), 339-352
- **MOHORIC et al.** *Bioresource Techn.,* 2009, vol. 100 (2), 851-858
- **ALTSCHUL, S.F. ; GISH, W. ; MILLER, W. ; MYERS, E.W. ; LIPMAN, D.J.** Basic local alignment search tool. *J. Mol. Biol.,* 1990, vol. 215, 403-410
- **ALTSCHUL, STEPHAN F. ; THOMAS L. MADDEN ; ALEJANDRO A. SCHAFFER ; JINGHUI ZHANG ; HHENG ZHANG ; WEBB MILLER ; DAVID J. LIPMAN.** Gapped BLAST and PSI-BLAST: a new generation of protein database search programs. *Nucleic Acids Res.,* 1997, vol. 25, 3389-3402
- **CHENNA et al.** Multiple sequence alignment with the Clustal series of programs. *Nucleic Acid Research,* 2003, vol. 31, 3497-3500
- **NOTREDAME et al.** T-Coffee: A novel method for multiple sequence alignments. *J. Mol. Biol.,* 2000, vol. 302, 205-217
- **BORASTON, A. B. et al.** *Biochem. J.,* 2004, vol. 382, 769-781

SEQUENCE LISTING

<110> Henkel AG & Co. KGaA

<120> Peroxidase mit Aktivität für Carotinoide

<130> PT031371

<150> 102013209545.7
<151> 2013-05-23

<160> 2

<170> PatentIn version 3.5

<210> 1
<211> 366
<212> PRT
<213> Bjerkandera adusta

<400> 1

```
Met Ala Phe Lys Gln Leu Ala Ala Ala Leu Ser Ile Ala Leu Ala Leu
1               5               10              15

Pro Phe Ser Gln Ala Ala Ile Thr Arg Arg Val Ala Cys Pro Asp Gly
            20              25              30

Val Asn Thr Ala Thr Asn Ala Ala Cys Cys Ala Leu Phe Ala Val Arg
            35              40              45

Asp Asp Ile Gln Gln Asn Leu Phe Asp Gly Gly Glu Cys Gly Glu Glu
    50              55              60

Val His Glu Ser Leu Arg Leu Thr Phe His Asp Ala Ile Gly Ile Ser
65              70              75              80

Pro Ser Leu Ala Ala Thr Gly Lys Phe Gly Gly Gly Gly Ala Asp Gly
            85              90              95

Ser Ile Met Ile Phe Asp Asp Ile Glu Pro Asn Phe His Ala Asn Asn
        100             105             110

Gly Val Asp Glu Ile Ile Asn Ala Gln Lys Pro Phe Val Ala Lys His
        115             120             125

Asn Met Thr Ala Gly Asp Phe Ile Gln Phe Ala Gly Ala Val Gly Val
    130             135             140

Ser Asn Cys Pro Gly Ala Pro Gln Leu Ser Phe Phe Leu Gly Arg Pro
145             150             155             160

Ala Ala Thr Gln Pro Ala Pro Asp Gly Leu Val Pro Glu Pro Phe Asp
```

```
                      165                      170                      175

      Ser Val Thr Asp Ile Leu Asn Arg Phe Ala Asp Ala Gly Gly Phe Thr
              180                      185                  190

      Thr Gln Glu Val Val Trp Leu Leu Ala Ser His Ser Ile Ala Ala Ala
              195                      200                  205

      Asp His Val Asp Pro Thr Ile Pro Gly Ser Pro Phe Asp Ser Thr Pro
          210                      215                  220

      Glu Ile Phe Asp Thr Gln Phe Phe Val Glu Thr Leu Leu Lys Gly Thr
      225                      230                  235                  240

      Leu Phe Pro Gly Thr Ser Gly Asn Gln Gly Glu Val Glu Ser Pro Leu
                      245                  250                  255

      Ala Gly Glu Ile Arg Leu Gln Ser Asp Ala Asp Phe Ala Arg Asp Ser
                  260                  265                  270

      Arg Thr Ala Cys Glu Trp Gln Ser Phe Val Asn Asn Gln Pro Arg Met
              275                  280                  285

      Gln Val Leu Phe Lys Ala Ala Met Gln Lys Leu Ser Ile Leu Gly His
          290                  295                  300

      Asp Leu Thr Gln Met Ile Asp Cys Ser Asp Val Ile Pro Val Pro Pro
      305                      310                      315                  320

      Ser Thr Ala Val Arg Gly Ser His Leu Pro Ala Gly Asn Thr Leu Asp
                      325                  330                  335

      Asp Ile Glu Gln Ala Cys Ala Ser Thr Pro Phe Pro Ser Leu Thr Ala
                  340                  345                  350

      Asp Pro Gly Pro Ala Thr Ser Val Ala Pro Val Pro Pro Ser
                  355                  360                  365
```

<210> 2
<211> 1101
<212> DNA
<213> Bjerkandera adusta

<400> 2

```
atggccttca agcaactcgc tgctgctctt tccatcgccc ttgctctccc cttctcgcaa      60

gctgcgatca ccagacgtgt ggcttgccca gatggcgtga acaccgcaac caacgcagcc     120

tgttgtgctt tgttcgccgt ccgtgatgac atccaacaga acttgttcga cggcggcgag     180


tgcggcgaag aagtgcacga gtcacttcga ctgaccttcc acgatgctat tggcatatct     240

ccaagccttg ccgccactgg caaattcggc ggcggaggtg ccgacgggtc tatcatgatc     300

ttcgacgaca tcgagcccaa cttccacgcc aacaacggcg tcgacgagat tatcaacgcg     360

cagaagccct tcgtggccaa gcacaacatg acggcaggcg actttattca attcgcaggc     420

gccgttggtg tgagcaactg ccctggtgct cctcaactga gcttcttcct cgggcgccct     480

gcagcgacgc agcccgcgcc tgacgggctt gttccggagc ccttcgactc ggtcaccgac     540

atcctcaatc gctttgccga tgctggcggc ttcacaaccc aagaagttgt ttggctcctt     600

gcctctcatt ccattgctgc cgctgaccac gtcgacccga cgatccctgg atcacccttc     660

gattctactc ccgaaatctt cgacacacag ttctttgttg agacgttgtt gaagggcacg     720

ttgttcccag gtacgagcgg caaccagggc gaagtcgagt ccccacttgc cggcgaaatt     780

cgtctccagt cagatgccga cttcgcacgt gactcgagga ctgcttgcga gtggcagtct     840

ttcgtcaata accagccccg gatgcaagtt ctgttcaagg cggctatgca gaagctgtct     900

atcttgggcc acgatctcac tcagatgatt gactgctccg acgtaatccc tgtacctccg     960

agcacagcgg tccgtggatc gcatctgcct gcgggcaaca cactggacga cattgaacag    1020

gcttgcgcct ccacgccatt cccctcgctc accgccgacc ctggtccggc cacctctgtt    1080

gcccctgtcc cgccttcgta a                                              1101
```